(19) Europäisches Patentamt
European Patent Office
Office européen des brevets

(11) EP 3 616 683 A1

(12) EUROPEAN PATENT APPLICATION
published in accordance with Art. 153(4) EPC

(43) Date of publication:
**04.03.2020 Bulletin 2020/10**

(21) Application number: **18791685.3**

(22) Date of filing: **07.03.2018**

(51) Int Cl.:
***A61K 8/86** (2006.01)* ***A61K 8/02** (2006.01)*
***A61K 8/44** (2006.01)* ***A61K 8/55** (2006.01)*
***A61Q 19/00** (2006.01)*

(86) International application number:
**PCT/JP2018/008766**

(87) International publication number:
**WO 2018/198541 (01.11.2018 Gazette 2018/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.04.2017 JP 2017090350**
**05.01.2018 JP 2018000680**

(71) Applicant: **Rohto Pharmaceutical Co., Ltd.**
**Osaka-shi**
**Osaka 544-8666 (JP)**

(72) Inventors:
- **FUJIMOTO, Junichi**
  **Osaka-shi**
  **Osaka 544-8666 (JP)**
- **PHAN, Phat Thanh**
  **Osaka-shi**
  **Osaka 544-8666 (JP)**

(74) Representative: **Cabinet Beau de Loménie**
**158, rue de l'Université**
**75340 Paris Cedex 07 (FR)**

# (54) COSMETIC COMPOSITION

(57) Provided is a cosmetic composition that maintains high stability and restrains from becoming cloud. This cosmetic composition is prepared by including (A) polyoxyethylene polyoxypropylene glycol, and (B) an amino acid based surfactant having, in a single molecule thereof, two or more hydrophobic groups and two or more hydrophilic groups. Furthermore, provided is a method of giving a cloudiness-restraining effect to a cosmetic composition, including causing the component (A) and the component (B) to coexist with each other in the cosmetic composition.

EP 3 616 683 A1

## Description

### TECHNICAL FIELD

The present invention relates to a cosmetic composition.

### BACKGROUND ART

Polyoxyethylene polyoxypropylene glycol (Pluronic surfactant) is a nonionic surfactant in which a triblock copolymer is composed of ethylene oxide (EO), which is hydrophilic, and propylene oxide (PO), which is hydrophobic; and has excellent properties such as low toxicity and biocompatibility. Thus, investigations have been made about the use of the surfactant in various fields of medical and pharmaceutical products, cosmetics, cleaners, and others.
According to Patent Document 1, an investigation is made about the use of polyoxyethylene polyoxypropylene glycol for eyewashes.
According to Patent Document 2, an investigation is made about the use of polyoxyethylene polyoxypropylene glycol for hair mousse.

### PRIOR ART DOCUMENTS

### PATENT DOCUMENTS

Patent Document 1: JP-A-2017-007995
Patent Document 2: JP-A-2000-204025

### SUMMARY OF THE INVENTION

### PROBLEMS TO BE SOLVED BY THE INVENTION

However, in the present situation, sufficient investigations have not been made about the storage stability of polyoxyethylene polyoxypropylene glycol in cosmetic compositions.
Thus, an object of the present invention is to provide a cosmetic composition in which polyoxyethylene polyoxypropylene glycol is improved in storage stability.

### MEANS FOR SOLVING THE PROBLEMS

The inventor has found out a new problem that when a cosmetic composition including (A) polyoxyethylene polyoxypropylene glycol contains a different component, the component (A) may become clouded/precipitate. The component (A) is a nonionic surfactant; thus, when this component becomes clouded/precipitates, an original surface activity thereof is unfavorably damaged. Thus, the inventor has paid attention to this problem to make eager investigations, so that the inventor has found out that when the component (A) is caused to coexist with (B) an amino acid based surfactant having, in a single molecule thereof, two or more hydrophobic groups and two or more hydrophilic groups in a cosmetic composition, the cosmetic composition can be restrained from becoming clouded/precipitating to be improved effectively in stability. Thus, the present invention has been accomplished.
Accordingly, the present invention provides the following:

[1] A cosmetic composition comprising:

(A) polyoxyethylene polyoxypropylene glycol, and
(B) an amino acid based surfactant having, in a single molecule thereof, two or more hydrophobic groups and two or more hydrophilic groups;

[2] The cosmetic composition according to item [1], wherein at least one of the hydrophobic groups in the component (B) has an acyl group;
[3] The cosmetic composition according to item [1] or [2], wherein each of the hydrophilic groups in the component (B) is at least one selected from the group consisting of a carboxyl group, a sulfonic acid group, a sulfate group, a phosphate group, and salts of these groups;

[4] The cosmetic composition according to any one of items [1] to [3], further comprising (C) a pharmaceutically acceptable base agent and/or additive;

[5] The cosmetic composition according to items [4], wherein the component (C) is at least one selected from the group consisting of antiseptic agents/preservatives, oxidation inhibitors, and anti-oxidants;

[6] The cosmetic composition according to any one of items [1] to [5], wherein a content of water is 10% or more by weight of a total of the cosmetic composition;

[7] The cosmetic composition according to any one of items [1] to [6], wherein the composition is at least one selected from the group consisting of lotion agents, gel agents, mist agents, and sheet agents (substrate-carried agents);

[8] The cosmetic composition according to any one of item [1] to [7], wherein the composition is a leave-on type; and the like.

The present invention can also provide, in another embodiment thereof, a method for giving a cloudiness-restraining effect to a cosmetic composition, comprising incorporating component (A) and component (B) into the cosmetic composition, wherein

the component (A) is polyoxyethylene polyoxypropylene glycol, and

the component (B) is an amino acid based surfactant having, in a single molecule thereof, two or more hydrophobic groups and two or more hydrophilic groups.

EFFECT OF THE INVENTION

In the cosmetic composition of the present invention, by incorporating, thereinto, (A) polyoxyethylene polyoxypropylene glycol and (B) an amino acid based surfactant having, in a single molecule thereof, two or more hydrophobic groups and two or more hydrophilic groups, the component (A) can be improved in stability to restrain the composition from becoming clouded.

MODE FOR CARRYING OUT THE INVENTION

[Cosmetic Composition]

The cosmetic composition of the present invention includes:

(A) polyoxyethylene polyoxypropylene glycol, and

(B) an amino acid based surfactant having, in a single molecule thereof, two or more hydrophobic groups and two or more hydrophilic groups.

[Component (A)]

Herein, polyoxyethylene polyoxypropylene glycol (component (A)) denotes a triblock copolymer represented by formula (1) illustrated below, which is composed of ethylene oxide units, and propylene oxide units. The component (A) is yielded by addition-polymerizing ethylene oxide to polypropylene glycol, which is yielded by addition-polymerizing propylene oxide molecules with each other.

[Formula 1]

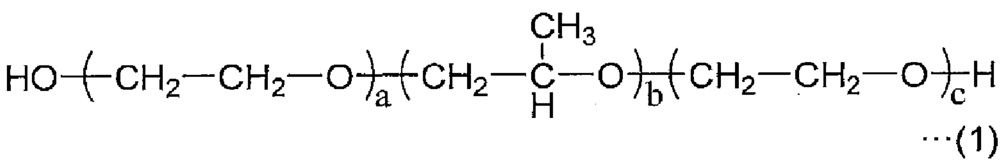

···(1)

In the formula (1), a, b and c each are any number other than zero.

The component (A) is not particularly limited about the molecular weight thereof, the average polymerization degree of the ethylene oxide units and that of the propylene oxide unit(s), and other factors thereof.

About the molecular weight of the component (A), the number-average molecular weight may be, for example, from 1000 to 30000, and is preferably from 1500 to 20000, more preferably from 2000 to 15000. The number-average molecular weight of the component (A) denotes the number-average molecular weight thereof in terms of the number-average molecular weight of polyethylene glycol, this molecular weight being measured by a gel permeation chromatography using a high performance liquid chromatography. The number-average molecular weight of the component (A) is measurable, for example, under conditions that a column therefor is a column (trade name: "Shodex OHpak SB-806M HQ, 8.0 x 300 mm", manufactured by Showa Denko K.K.), the temperature of the column is room temperature, a mobile phase thereof is water, the flow rate is 1.0 mL/minute, and a detector therefor is an RI.

In the formula (1), the average polymerization degree of the propylene oxide units of the component (A), which is represented by b, may be, for example, from 5 to 100, and is preferably from 10 to 80, more preferably from 15 to 70, even more preferably from 20 to 60, in particular preferably from 22 to 50, most preferably from 25 to 40.

The average polymerization degree of ethylene oxide units of component (A), which are represented by a and c in the formula (1), in total, for example, from 2 to 200, and is preferably from 5 to 150, more preferably from 10 to 100, even more preferably from 15 to 70, in particular preferably from 20 to 50, most preferably from 22 to 30.

Examples of this component (A) include polyoxyethylene polyoxypropylene glycol (19 E.O.) (21 P.O.), polyoxyethylene polyoxypropylene glycol (16 E.O.) (30 P.O.) (poloxamer (182), polyoxyethylene polyoxypropylene glycol (26 E.O.) (30 P.O.) (poloxamer (184), polyoxyethylene polyoxypropylene glycol (38 E.O.) (30 P.O.) (poloxamer 185), polyoxyethylene polyoxypropylene glycol (150 E.O.) (30 P.O.) (poloxamer (188), polyoxyethylene polyoxypropylene glycol (48 E.O.) (35 P.O.) (poloxamer (215), polyoxyethylene polyoxypropylene glycol (54 E.O.) (39 P.O.) (poloxamer (235), polyoxyethylene polyoxypropylene glycol (40 E.O.) (54 P.O.) (poloxamer 333), polyoxyethylene polyoxypropylene glycol (196 E.O.) (67 P.O.) (poloxamer (407), polyoxyethylene polyoxypropylene glycol (200 E.O.) (70 P.O.), polyoxyethylene polyoxypropylene glycol (5 E.O.) (30 P.O.), polyoxyethylene polyoxypropylene glycol (10 E.O.) (30 P.O.) polyoxyethylene polyoxypropylene glycol (25 E.O.) (30 P.O.), polyoxyethylene polyoxypropylene glycol (160 E.O.) (30 P.O.), polyoxyethylene polyoxypropylene glycol (5 E.O.) (35 P.O.), polyoxyethylene polyoxypropylene glycol 30 E.O.) (35 P.O.), polyoxyethylene polyoxypropylene glycol (150 E.O.) (35 P.O.), and polyoxyethylene polyoxypropylene glycol (300 E.O.) (55 P.O.). Herein, POE is polyoxyethylene and POP is polyoxypropylene; and the number in each of the parenthesis pairs may be described as the number of the additional mole of ethylene oxide or propylene oxide.

From the viewpoint of producing the advantageous effects of the present invention remarkably, among such components (A), more preferred is/are polyoxyethylene polyoxypropylene glycol (26 E.O.) (30 P.O.) (poloxamer 184) and/or polyoxyethylene polyoxypropylene glycol (25 E.O.) (30 P.O.).

The content of the component (A) can be appropriately set in accordance with the species of the component (A), the species and the amounts of the other components, the agent-formulation form of the cosmetic composition, and others, and is not limited. From the viewpoint of producing the advantageous effects of the present invention remarkably, the content of the component (A) may be, for example, 0.01% or more by weight of a total of the cosmetic composition, and is preferably 0.05% or more, more preferably 0.1% or more, even more preferably 0.3% or more, in particular preferably 0.5% or more by weight thereof. The content of the component (A) may be, for example, 50% or less by weight of a total of the cosmetic composition, and is preferably 40% or less, more preferably 30% or less, even more preferably 20% or less, even more preferably 10% or less, in particular preferably 5% or less by weight thereof. The content of the component (A) may be, for example, from 0.01 to 50% by weight of a total of the cosmetic composition, and is preferably from 0.05 to 40%, more preferably from 0.1 to 30%, even more preferably from 0.3 to 20%, even more preferably from 0.5 to 10%, in particular preferably from 0.5 to 5% by weight thereof.

[Component (B)]

The component (B), that is an amino acid based surfactant having, in a single molecule thereof, two or more hydrophobic groups and two or more hydrophilic groups, is an amphipathic gemini type (double chain type, twin type) surfactant, and is, for example, a product "Pellicer" (manufactured by Asahi Kasei Chemicals Corp.).

The two or more hydrophobic groups, which the component (B) has in a single molecule thereof, each are not limited, but may be independently a linear, branched or cyclic saturated or unsaturated chain having 2 to 20 carbon atoms.

The two or more hydrophilic groups, which the component (B) has in a single molecule thereof, are not limited, and may be each independently, for example, a carboxyl group, a sulfonic acid group, a sulfuric acid residue, a phosphoric acid residue or a salt thereof; an oxyalkylene group or a polyethylene glycol group; or an amino group, a quaternary ammonium group, a pyridinium group, a sulfonium group, or a salt thereof.

The number of the hydrophobic groups or that of the hydrophilic groups, which the component (B) has in a single molecule thereof, is not particularly limited; and may be from 2 to 60 both inclusive, and is preferably from 2 to 40 both inclusive, more preferably from 2 to 20 both inclusive. The number of the hydrophilic groups is preferably from 3 to 10 both inclusive although the number is not limited.

Examples of the hydrophobic groups in the component (B) include n-acetyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, and n-eicosyl residues; and branched chain isomers thereof, as well as unsaturated residues which correspond to these hydrophobic groups and which each have, at one, two or three positions thereof, one or more unsaturated moieties.

The two or more hydrophobic groups in the component (B) each are not limited, but preferably are an acyl group derived from a saturated or unsaturated fatty acid having 2 to 20 carbon atoms. When the component (B) is rendered an acyl compound having two or more acyl groups, the component (B) becomes good in solubility in water. When the component (B) has two or more acyl groups, the acyl groups may be each independently from each other to be different from each other or be the same. The acyl groups each are preferably a group derived from a saturated or unsaturated fatty acid having 2 to 20 carbon atoms, and may be linear, branched or cyclic. However, the acyl groups do not include, in the category, any group that has been turned to a carboxyl group.

The acyl groups are not particularly limited, and may each be an acyl group derived from an fatty acid, examples of this acid including acetic acid,, propionic acid, butyric acid, pentanoic acid, hexanoic acid, heptanoic acid, caprylic acid, pelargonic acid, capric acid, undecanoic acid, lauric acid, tridecanoic acid, myristic acid, pentadecanoic acid, palmitic acid, margaric acid, stearic acid, nonadecanoic acid, arachic acid, and other linear fatty acids; 2-butyl-5-methylpentanoic acid, 2-isobutyl-5-methylpentanoic acid, dimethyloctanoic acid, dimethylnonanoic acid, 2-butyl-5-methylhexanoic acid, methylundecanoic acid, dimethyldecanoic acid, 2-ethyl-3-methylnonanoic acid, 2,2-dimethyl-4-ethyloctanoic acid, methyldocosanoic acid, 2-propyl-3-methylnonanoic acid, methyltridecanoic acid, dimethyldodecanoic acid, 2-butyl-3-methylnonanoic acid, methyltetradecanoic acid, ethyltridecanoic acid, propyldodecanoic acid, butylundecanoic acid, pentyldecanoic acid, hexylnonanoic acid, 2-(3-methylbutyl)-3-methylnonanoic acid, 2-(2-methylbuty))-3-methylnonanoic acid, butylethylnonanoic acid, methylpentadecanoic acid, ethyltetradecanoic acid, propyltridecanoic acid, butyldodecanoic acid, pentylundecanoic acid, hexyldecanoic acid, heptylnonanoic acid, dimethyltetradecanoic acid, butylpentylheptanoic acid, trimethyltridecanoic acid, methylhexadecanoic acid, ethylpentadecanoic acid, propyltetradecanoic acid, butyltridecanoic acid, pentyldodecanoic acid, hexylundecanoic acid, heptyldecanoic acid, methylheptylnonanoic acid, dipentylheptanoic acid, methylheptadecanoic acid, ethylhexadecanoic acid, ethylhexadecanoic acid, propylpentadecanoic acid, butyltetradecanoic acid, pentyltridecanoic acid, hexyldodecanoic acid, heptylundecanoic acid, octyldecanoic acid, dimethylhexadecanoic acid, methyloctylnonanoic acid, methyloctadecanoic acid, ethylheptadecanoic acid, dimethylheptadecanoic acid, methyloctyldecanoic acid,, methylnonadecanoic acid, methylnonadecanoic acid, dimethyloctadecanoic acid, butylheptylnonanoic acid; and other branched fatty acids; octene acid, nonenoic acid, decenoic acid, caproleic acid, undecylenic acid, linderic acid, obtusilic acid, lauroleic acid, tridecenoic acid, tsuzuic acid, myristoleic acid, pentadecenoic acid, hexedecenoic acid, palmitoleic acid, heptadecenoic acid, octadecenoic acid, oleic acid, nonadecenoic acid, gondoic acid, and other linear mono-ene acids; methylheptenoic acid, methylnonenoic acid, methylundecenoic acid, dimethyldecenoic acid, methyldodecenoic acid, methyltridecenoic acid, dimethyldodecenoic acid, dimethyltridecenoic acid, methyloctadecenoic acid, dimethylheptadecenoic acid, ethyloctadecenoic acid, and other branched mon-ene acids; linoleic acid, linoelaidic acid, eleostearic acid, linolenic acid, linolenic elaidic acid, pseudo eleostearic acid, parinaric acid, arachidonic acid, and other di- or tri-ene acids; octynoic acid, nonynoic acid, decynoic acid, undecynoic acid, dodecynoic acid, tridecynoic acid, tetradecynoic acid, pentadecynoic acid, heptadecynoic acid, octadecynoic acid, nonadecynoic acid, dimethyloctadecynoic acid, other acetylenic acids; methyleneoctadecenoic acid, methyleneoctadecanoic acid, Aleprolic acid, aleprestic acid, aleprylic acid, alepric acid, hydnocarpic acid, shoal muglinic acid, gorlic acid, α-cyclopentyl acid, α-cyclohexyl acid, α-cyclopentylethyl acid, and other cyclic acids, and other fatty acids.

The acyl groups may be acyl groups derived from fatty acids yielded from natural fat and oil, and are preferably acyl groups derived from mixed fatty acids containing 80% or more of the above-mentioned saturated or unsaturated fatty acid(s) having 2 to 20 carbon atoms. The acyl groups are, for example, acyl groups derived from coconut oil fatty acid, palm oil fatty acid, linseed oil fatty acid, sunflower oil fatty acid, soybean oil fatty acid, sesame oil fatty acid, castor oil fatty acid, olive oil fatty acid, camellia oil fatty acid, rapeseed oil fatty acid, palm kernel oil fatty acid, and other acids. These acyl compounds, which each have an acyl group, may be used in any combination of two or more thereof. The acyl groups each are preferably an acyl group derived from a saturated or unsaturated fatty acid having 8 to 20 carbon atoms.

Furthermore, from the viewpoint of producing the advantageous effects of the present invention remarkably, the component (B) is more preferably a compound represented by the following formula (2), in particular preferably a compound represented by formula (3) illustrated below:

[Formula 2]

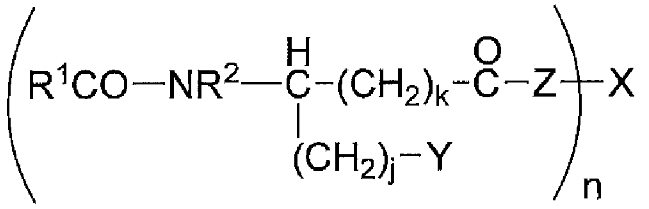

···(2)

In the formula (2), $R^1$ is a hydrocarbon group having 1 to 23 carbon atoms. $R^1$ is preferably a hydrocarbon group having 7 to 17 carbon atoms. $R^1$ may be any one of linear chains, branched chains, cyclic chains, and aromatic hydrocarbon chains, and may have a substituent group.

In the formula (2), $R^2$ is hydrogen or a hydrocarbon group having 1 to 3 carbon atoms. $R^2$ may have a carboxyl group or a sulfonic acid group. Examples of the hydrocarbon group having 1 to 3 carbon atoms include methyl, ethyl, propyl, isopropyl, hydroxymethyl, hydroxyethyl, hydroxy(iso)propyl, dihydroxy(iso)propyl, carboxymethyl, carboxyethyl, carboxypropyl, and sulfoethyl groups. $R^2$ is preferably hydrogen.

In the formula (2), Y is a carboxyl group, sulfonic acid group, sulfate group or phosphate group, or a salt of any one of these groups. Y is preferably a carboxyl group or a salt thereof.

Y may be combined with a basic substance that may be of various types to form a salt. A metal which can form the salt is not limited, and examples thereof include alkali metals such as sodium, potassium and lithium; alkaline earth metals such as calcium and magnesium; and aluminum, zinc, iron, cobalt, titanium, zirconium, and silver.

The basic substance, which can form the salt, is not limited, and examples thereof include ammonia; monoethanolamine, diethanolamine, triethanolamine, triisopropanolamine, and other organic amines; and arginine, lysine and other basic amino acids. Other examples thereof include ammonium salts, and polyvalent metal salts.

In the formula (2), recurred Y may include two or more salt species selected at will from the above-mentioned salts.

In the formula (2), -Z- is -NR'- wherein R' is hydrogen or a hydrocarbon group having 1 to 10, -O-, or -S-.

In the formula (2), X is a hydrocarbon chain having a molecular weight of 1000000 or less. X may be a linear chain, a branched chain, a cyclic chain or an aromatic hydrocarbon chain. X may have a substituent, and in particular preferably has a carboxyl group. The number of carbon atoms in X is preferably from 1 to 40, and the molecular weight thereof is preferably from 14 to 2000.

When X contains, for example, a carboxyl group, a sulfonic acid group, a sulfate group and a phosphate group, the groups may be combined with various basic substances to form salts. Examples of a metal which may form the salts, and the basic substance containing the metal include the same salts as described above.

In the formula (2), the recurring number of a moiety in the parentheses is n, and the recurred moieties may be the same or different. n is an integer of 2 to 20. Preferably, n is 2. Moreover, j and k each are any one of 0, 1 and 2, and j and k are not simultaneously zero.

A compound represented by the formula (3) illustrated below is an example of the compound represented by the formula (2) in which n is 2.

[Formula 3]

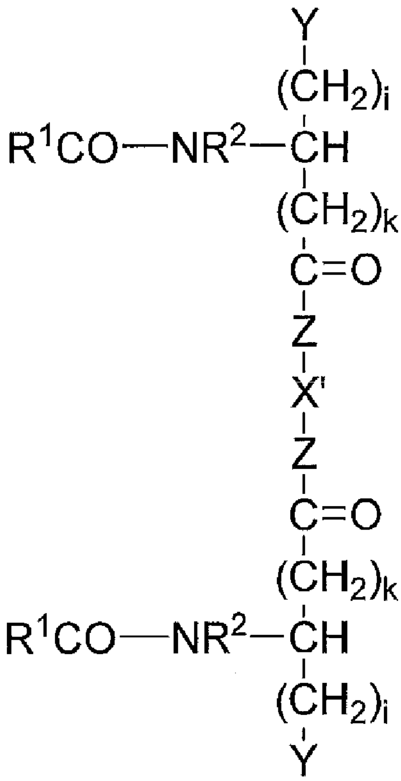

···(3)

In the formula (3), $R^1$, $R^2$, Y, -Z-, j and k are the same as in the formula (2).

In the formula (3), X' represents a hydrocarbon chain having 1 to 20 carbon atoms and having at least one selected from the group consisting of a carboxyl group or salts; -NHR' group wherein R' is hydrogen or a hydrocarbon group having 1 to 10 carbon atoms; a -OH group; and a -SH group.

In the formula (3), X' is preferably a hydrocarbon chain having 1 to 20 carbon atoms and having a carboxyl group or a salt; $R^2$ each are preferably hydrogen; Y each are preferably a carboxyl group or a salt thereof; and Z each are preferably -NH-. It is preferred that j and k each are 0 or 2, and j and k are not simultaneously zero.

From the viewpoint of producing the advantageous effects of the present invention remarkably, the component (B) is preferably at least one selected from the group consisting of dilauramidoglutamide lysine, and salts thereof although preferred examples of the component (B) are not limited. Preferred one of the salts of dilauramidoglutamide lysine is a sodium salt thereof.

The content of the component (B) may be appropriately set in accordance with the species of the component (B), the species and the amounts of the components, the agent-formulation form of the cosmetic composition, and others, and is not limited. From the viewpoint of producing the advantageous effects of the present invention remarkably, the content may be, for example, 0.001% or more by weight of a total of the cosmetic composition, and is preferably 0.005% or more, more preferably 0.01% or more, even more preferably 0.03% or more, in particular preferably 0.05% or more by weight thereof. The content of the component (B) may be, for example, 30% or less by weight of a total of the cosmetic composition, and is preferably 20% or less, more preferably 10% or less, even more preferably 5% or less, in particular preferably 1% or less by weight thereof. The content of the component (B) may be, for example, from 0.01 to 30% by weight of a total of the cosmetic composition, and is preferably from 0.05 to 20%, more preferably from 0.05 to 10%, even more preferably from 0.05 to 5%, in particular preferably from 0.05 to 1% by weight thereof.

The blend ratio between the component (A) and the component (B) may be appropriately set in accordance with the species of the component (A) and the component (B), the species and the amount of other components, and dosage form. From the viewpoint of producing the advantageous effects of the present invention remarkably, the total amount of the component (B) may be from 0.001 to 210 parts by weight for 100 parts by weight of the component (A), and is preferably from 0.005 to 60 parts, more preferably from 0.01 to 30 parts, even more preferably from 0.05 to 20 parts, in particular from 0.1 to 18 parts, most preferably from 1 to 15 parts by weight therefor.

[Component (C)]

The cosmetic composition of the present invention preferably further contains component (C), a pharmaceutically acceptable base agent and/or additive. Even when the stability of the component (A) is damaged by the coexistence

of the component (A) with the component (C) in the cosmetic composition, the stability of the component (A) can be kept by the matter that this composition further contains the component (B) . The cosmetic composition of the present invention can be made into a drug by mixing the composition with a known base agent and carrier. In addition, additives may be blended into the cosmetic compositions of the present invention. Examples of the additives include other surfactants, a viscosity adjuster, an oil component, an alcohol, a thickener, an antiseptic agent/preservative, an anti-oxidant, an oxidation inhibitor, a chelating agent, a pH adjuster, a stabilizing agent, a solubilizing agent, a suspending agent, an isotonic agent, a buffering agent, soothing agent, a dispersant, a perfume, a pigment, a colorant, and water. These additives may be used alone or in any combination of two or more thereof. The component (C) is not limited, and is preferably, for example, at last one selected from the group consisting of antiseptic agents/preservatives, oxidation inhibitors, and anti-oxidants.

Examples of the base agent or carrier include liquid paraffin, squalane, gelatinized hydrocarbons (such as Plastibase), ozokerite, α-olefin oligomer, light liquid paraffin, and other hydrocarbons; methylpolysiloxane, crosslinked methylpolysiloxane, highly polymerized methylpolysiloxane, cyclic silicone, alkyl-modified silicone, crosslinked alkyl-modified silicone, amino-modified silicone, polyether-modified silicone, polyglycerin-modified silicone, crosslinked polyether-modified silicone, crosslinked alkyl-polyether-modified silicone, silicone/alkyl-chain co-modified polyether modified silicone, silicone/alkyl-chain co-modified polyglycerin modified silicone, polyether-modified branched silicone, polyglycerin-modified branched silicone, acrylic silicone, phenyl-modified silicone, silicone resin, and other silicone oils; polyethylene glycol; dioxane; isopropyl myristate, octyldodecyl myristate, isopropyl palmitate, cetyl palmitate, isononyl isononanoate, pentaerythrite tetra 2-ethylhexanoate, and other esters; ethanol, isopropanol, and other lower alcohols; diethylene glycol monomethyl ether, diethylene glycol monoethyl ether, and other glycol ethers; polyethylene glycol, propylene glycol, 1,3-butylene glycol, glycerin, isoprene glycol, and other polyhydric alcohols; and water, and other water-based base agents. From the viewpoint of producing the advantageous effects of the present invention remarkably, the base agent or carrier is preferably dipropylene glycol, 1,3-butylene glycol, or glycerin.

Such base agents or carries may be used alone or in any combination of two or more thereof.

Examples of the other surfactants include sorbitan monoisostearate, sorbitan monolaurate, sorbitan monopalmitate, sorbitan monostearate, diglycerol sorbitan penta-2-ethylhexylate, diglycerol sorbitan tetra-2-ethylhexylate, and other sorbitan fatty acid esters; propylene glycol monostearate, and other propylene glycol fatty acid esters; polyoxyethylene hydrogenated castor oil 40 (HCO-40), polyoxyethylene hydrogenated castor oil 50 (HCO-50), polyoxyethylene hydrogenated castor oil 60 (HCO-60), polyoxyethylene hydrogenated castor oil 80, and other hydrogenated castor oil derivatives; polyoxyethylene (20) sorbitan monolaurate (polysorbate 20), polyoxyethylene (20) sorbitan monostearate (polysorbate 60), polyoxyethylene (20) sorbitan monooleate (polysorbate 80), polyoxyethylene (20) sorbitan isostearate, and other polyoxyethylene sorbitan fatty acid esters; polyoxyethylene moncoconut oil fatty acid glyceryl; glycerin alkyl ether; alkyl glucoside; polyoxyethylene (20) oleyl ether, polyoxyethylene cetyl ether, and other polyoxyalkylene alkyl ethers; stearylamine, oleylamine, and other amines; polyoxyethylene/methyl polysiloxane copolymer, lauryl PEG-9 polydimethylsiloxyethyldimethicone, PEG-9 polydimethylsiloxyethyldimethicone, and other silicone surfactants; lauric acid salts, palmitic acid salts, cocoylglutamic acid salts, coconut oil methylalanine salts, acylmethyltaurine salts, polyoxyethylene lauryl sulfates, and other anionic surfactants; lauryldiaminoethylglycine salt, coconut oil fatty acid betaine salts, and other amphoteric surfactants; polyoxyethylene lauryl alcohol ether, and other nonionic surfactants; and stearyltrimethylammonium chloride, and other cationic surfactants.

Examples of the viscosity adjuster include xanthan gum, carboxymethylcellulose, hydroxyethylcellulose, partially crosslinked polyacrylic acid, montmorillonite, hectorite, saponite, vermiculite, nontrorite, zauconite, laponite, and other thickeners; and propylene glycol, ethanol, propanol, isopropanol, ethylene glycol, polyethylene glycol, polypropylene glycol, glycerin, polyglycerin, sorbitol, pentaerythritol, benzenesulfonic acid salts, lower alkylbenzenesulfonic acid salts each having 1 to 4 carbon atoms, and other viscosity reducers.

Examples of the oil component include natural animal/vegetable oils and fats, hydrocarbon oils, ester oils, silicone oils, higher alcohols, higher fatty acids, and animal/vegetable and synthetic essential oils.

Examples of the natural anima/vegetable oils and fats include avocado oil, linseed oil, almond oil, olive oil, cocoa oil, beef tallow, tung oil, wheat germ oil, sesame oil, rice germ oil, rice bran oil, safflower oil, soybean oil, evening primrose oil, camellia oil, corn oil, rapeseed oil, horse fat, persick oil, palm oil, palm kernel oil, castor oil, sunflower oil, pig fat, grape oil, jojoba oil, macadamianut oil, mink oil, cottonseed oil, Japan wax, coconut oil, hardened coconut oil, peanut oil, lanolin, yolk oil, and rose hip oil.

The hydrocarbon oils may each be a paraffinic hydrocarbon, or an olefinic hydrocarbon. Examples thereof include squalane, squalene, ceresin, paraffin, pristane, microcrystalline wax, liquid paraffin, and vaseline.

The ester oils may each be a synthetic ester, or an ester made from a higher alcohol and a higher fatty acid. Examples thereof include diisobutyl adipate, 2-hexyldecyl adipate, di-2-heptylundecyl adipate, isostearyl isostearate, trimethylolpropane triisostearate, cetyl 2-ethylhexanoate, neopentyl glycol di-2-ethylhexanoate, trimethylolpropane tri-2-ethylhexanoate, pentaerythritol tetra-2-ethylhexanoate, cetyl octanoate, oleyl oleate, octyldodecyl oleate, decyl oleate, neopentyl glycol dicaprate, 2-ethylhexyl succinate, isocetyl stearate, butyl stearate, diisopropyl sebacate, cetyl lactate,

tetradecyl lactate, isopropyl myristate, octyldodecyl myristate, cetyl myristate, myristyl myristate, octyl palmitate, 2-ethylhexyl palmitate, 2-hexyldecyl palmitate, 2-heptylundecyl palmitate, cholesteryl 12-hydroxystearate, phytosteryl oleate, diisostearyl malate, p-methoxycinnamate, and tetrarosin acid pentaerythrite ester.

Examples of the silicone oils include dimethylpolysiloxane, highly polymerized methylpolysiloxane, methylphenylpolysiloxane, methyl hydrogenpolysiloxane, octamethylcyclotetrasiloxane, octamethylcyclopentasiloxane, decamethylcyclohexanesiloxane, stearoxysilicone, and other higher-alkoxy-modified silicones; alkyl-modified silicones; and higher-fatty-acid-ester-modified silicones.

Examples of the higher alcohols include octyldodecanol, isostearyl alcohol, oleyl alcohol, stearyl alcohol, cetanol, and behenyl alcohol.

The higher fatty acids may each be a linear or branched chain saturated or unsaturated fatty acid having 12 to 22 carbon atoms. Examples thereof include isostearic acid, oxystearic acid, oleic acid, stearic acid, palmitic acid, behenic acid, myristic acid, lauric acid, lanolin acid, linolic acid, and linolenic acid.

Examples of the thickener include guar gum, locust bean gum, carrageenan, xanthan gum, carboxymethylcellulose, hydroxymethylcellulose, hydroxyethylcellulose, hydroxypropylcellulose, hydroxypropylmethylcellulose, hydrophobically-modified hydroxypropylmethylcellulose, polyvinyl alcohol, polyvinyl pyrrolidone, carboxyvinyl polymer, alkyl acrylate methacrylate copolymer, polyethylene glycol, bentonite, (hydroxyethyl acrylate/Na acryloyldimethyltaurine) copolymer, and (ammonium acryloyldimethyltaurine/vinylpyrrolidone) copolymers.

Examples of the antiseptic agent/preservative include isopropylmethylphenol, chlorobutanol, benzyl alcohol, phenethyl alcohol, benzoic acid, sodium benzoate, dehydroacetic acid, sodium dehydroacetate, isobutyl p-oxybenzoate, isopropyl p-oxybenzoate, butyl p-oxybenzoate, ethyl p-oxybenzoate, propyl p-oxybenzoate, benzyl p-oxybenzoate, methyl p-oxybenzoate (methylparaben), phenoxyethanol, ethylhexylglycerin, 1,2-pentanediol, 1,2-hexanediol, and glyceryl caprate. From the viewpoint of producing the advantageous effects of the present invention remarkably, the antiseptic agent/preservative is preferably phenoxyethanol, methyl p-oxybenzoate (methyl paraben), ethyl p-oxybenzoate, and propyl p-oxybenzoate. The content of the antiseptic agent/preservative may be, for example, from 0.001 to 1% by weight of a total of the cosmetic composition.

Examples of the anti-oxidant include dibutylhydroxytoluene (BHT), butylhydroxyanisole, sorbic acid, sodium sulfite, ascorbic acid, erythorbic acid, and L-cysteine hydrochloride. The content of the anti-oxidant may, for example, from 0.001 to 1% by weight of a total of the cosmetic composition.

Preferred examples of the anti-oxidant include sulfites, and ascorbic acid. The content of the anti-oxidant may be, for example, from 0.001 to 1% by weight of a total of the cosmetic composition.

Examples of the chelating agent include an EDTA/disodium salt, and an EDTA/calcium/disodium salt.

Example of the pH adjuster include inorganic acids (such as hydrochloric acid, and sulfuric acid), organic acids (such as lactic acid, sodium lactate, citric acid, sodium citrate, succinic acid, and sodium succinate), inorganic bases (such as potassium hydroxide, and sodium hydroxide), and organic bases (such as triethanolamine, diisopropanolamine, and triisopropanolamine).

Examples of the stabilizer include sodium polyacrylate, dibutylhydroxytoluene, and butylhydroxyanisole.

Preferred examples of the solubilizing agent include polyethylene glycol, propylene glycol, D-mannitol, benzyl benzoate, ethanol, trisaminomethane, cholesterol, triethanolamine, sodium carbonate, and sodium citrate.

Preferred examples of the suspending agent include stearyl triethanolamine, sodium laurylsulfate, laurylaminopropionic acid, lecithin, benzalkonium chloride, benzethonium chloride, glycerin monostearate and other surfactants; and polyvinyl alcohol, polyvinyl pyrrolidone, sodium carboxymethylcellulose, methylcellulose, hydroxymethylcellulose, hydroxyethylcellulose, hydroxypropylcellulose and other hydrophilic polymers.

Preferred examples of the isotonic agent include sodium chloride, glycerin, and D-mannitol.

Preferred examples of the buffering agent include phosphates, acetates, carbonates, citrates, and other buffer solutions.

A preferred example of the soothing agent is benzyl alcohol.

Examples of the dispersant include sodium pyrophosphate, sodium hexametaphosphate, polyvinyl alcohol, polyvinyl pyrrolidone, methyl vinyl ether/maleic anhydride crosslinked copolymers, and organic acids.

Examples of the pigment include inorganic pigments and natural colorants.

As far as the advantageous effects of the present invention are not damaged, the cosmetic composition of the present invention may contain any other effective component. Specific examples of the organic component include a moisturizing component, a pearl gloss imparting agent, a conditioning agent, a scrub agent, a blood circulation promoter, an astringent component, an ultraviolet absorbing component, an ultraviolet scattering component, a cleaning component, an antibacterial component, an anti-inflammatory agent, a vitamin, a peptide or any derivative thereof, an amino acid or any derivative thereof, and a cell activating component.

Examples of the moisturizing component include glycerin, 1,3-butylene glycol, dipropylene glycol, propylene glycol, polyethylene glycol, diglycerin, and other polyhydric alcohols; trehalose, xylitol, oligosaccharide, and other saccharides; hyaluronic acid and analogs, heparin analogs, sodium chondroitin sulfate, collagen, elastin, keratin, chitin,

chitosan, and other polymeric compounds; glycine, aspartic acid, arginine, and other amino acids; sodium lactate, urea, sodium pyrrolidonecarboxylate, and other natural moisturizing factors; ceramide, cholesterol, phospholipid, and other lipids; and chamomilla recutita extract, hamamelis extract, tea leaf extract, perilla extract, coix seed (pearl barley) extract, and other plant extracts. From the viewpoint of producing the advantageous effect of the present invention stably, the moisturizing component is preferably 1,3-butylene glycol, glycerin or dipropylene glycol.

The cosmetic composition of the present invention preferably contains, as its other effective component, the moisturizing agent to maintain the moisture of the skin to be effectively used. The moisturizing component is appropriately changed in accordance with the species and the content of the components (A) and (B), the species and the contents of the other components, the formulation agent-formulation form of the cosmetic composition, and others, and is not limited. The moisturizing agent is preferably at least one selected from the group consisting of hyaluronic acid and analogs thereof, sodium chondroitinsulfate, collagen, and elastin; more preferably at least one selected from the group consisting of hyaluronic acid and analogs thereof, sodium chondroitinsulfate, and collagen; even more preferably hyaluronic acid or any analog thereof, and/or sodium chondroitin sulfate; in particular preferably hyaluronic acid or any analog thereof.

Examples of hyaluronic acid or the analog include hyaluronic acid, any derivative thereof, and salts of these compounds. Hyaluronic acid is an acidic mucopolysaccharide species, and is a polysaccharide including, as constituent units thereof, two saccharides of glucuronic acid and N-acetylglucosamine. Hyaluronic acid can be extracted and collected from animal tissues such as cockscombs, skins of sharks, umbilical cords, eyeballs, skins and cartilages; hyaluronic-acid-producing microorganisms such as microorganism in the genus streptococci; and preparations of animal cells or plant cells. A commercially available product thereof may be bought.

It is generally said that hyaluronic acid obtained by extracting animal tissues, microorganisms and others as described above has an average molecular weight of 1000 kDa or more. It is known that hyaluronic acid is a very high molecular-weight molecule. In the present invention, such a high-molecular-weight hyaluronic acid may be used, or a low-molecular-weight hyaluronic acid yielded by decomposing a high-molecular-weight hyaluronic acid may be used. The low-molecular-weight hyaluronic acid can be yielded by, for example, a method of hydrolyzing a high-molecular-weight hyaluronic acid in the presence of an acid such as hydrochloric acid, or an alkali, a method of using an enzyme such as hyaluronidase to treat a high-molecular-weight hyaluronic acid, or a method of cutting a high-molecular-weight hyaluronic acid physically by ultrasonic waves or shearing. The low-molecular-weight hyaluronic acid may also be a bought commercially available product thereof. The cosmetic composition of the present invention preferably makes use of any low-molecular-weight hyaluronic acid although the invention is not limited to this embodiment.

Herein, the average molecular weight of hyaluronic acid is not particularly limited as far as the advantageous effects of the invention are produced. The molecular weight may be, for example, from about 0.1 to 1800 kDa. In order that the advantageous effects of the invention can be more effectively produced, the average molecular weight of hyaluronic acid is preferably from about 0.1 to 700 kDa, more preferably from about 0.2 to 600 kDa, even more preferably from about 4 to 500 kDa, even more preferably from about 5 to 400 kDa, in particular preferably from about 8 to 350 kDa.

Herein, the wording "average molecular weight" denotes "weight-average molecular weight". The weight-average molecular weight can be gained by a known measuring method. Specifically, the weight-average molecular weight is measurable by size exclusion chromatography. Conditions therefor are as follows: column: TSKgel GMPWXL, 7.8 mm $\times$ 30 cm (manufactured by Tosoh Corp.); column temperature: a constant temperature around about 40°C; detector: a differential refractometer; mobile phase: 0.2 mol/L NaCl; flow rate: 0.3 mL/minute; injected amount of a sample: 100 $\mu$L; and standard substance: pullulan standard product (STANDARD P-82, manufactured by Showa Denko K.K.).

The derivative of hyaluronic acid is not particularly limited as far as the derivative is pharmaceutically or physiologically allowable. Examples thereof include acetylated hyaluronic acid, in which a hydroxyl group has been acetylated, sulfated hyaluronic acid, in which a hydroxyl group has been sulfated, cationized hyaluronic acid, which has been cationized, hydrophobically-modified hydrolyzed hyaluronic acid (such as alky (C12-13) glyceryl hydrolyzed hyaluronate), hyaluronic acid cross polymer, and propylene glycol hyaluronate. Preferred examples of the derivative of hyaluronic acid include acetylated hyaluronic acid, cationized hyaluronic acid, hydrolyzed hyaluronic acid, hyaluronic acid cross polymer and propylene glycol hyaluronate.

The salts of hyaluronic acid or the derivative thereof are not particularly limited, either as far as the salts are pharmaceutically or physiologically allowable. Examples thereof include salts of alkali metals such as sodium and potassium; salts of alkaline earth metals such as calcium and magnesium; and salts of other metals such as aluminum. The salts of hyaluronic acid or the derivative thereof are preferably sodium and potassium salts thereof. Specific examples of the salts of hyaluronic acid or the derivative thereof include sodium hyaluronate, potassium hyaluronate, calcium hyaluronate, magnesium hyaluronate, zinc hyaluronate, ammonium hyaluronate, monoethanolamine hyaluronate, sodium acetylated hyaluronate, potassium acetylated hyaluronate, calcium acetylated hyaluronate, magnesium acetylated hyaluronate, zinc acetylated hyaluronate, ammonium acetylated hyaluronate, sodium hyaluronic acid cross polymer, and hydroxypropyltriammonium hyaluronate.

In the cosmetic composition of the present invention, hyaluronic acid, derivatives thereof, and salts of these

compounds may be used alone or in any combination of two or more thereof. The cosmetic composition of the invention preferably contains at least two selected from the group consisting of the following to maintain the moisture of the skin to be effectively used: hyaluronic acid, acetylated hyaluronic acid, hydrolyzed hyaluronic acid, cationized hyaluronic acid, hyaluronic acid cross polymer, and salts of these compounds.

Hyaluronic acid, derivatives thereof, and salts thereof that are used in the present invention may be commercially available products. Examples of hyaluronic acid include products of a product name "SODIUM HYALURONATE HA 12N" (Shiseido Co., Ltd.), an average molecular weight of 1100 to 1600 kDa; a product name "HYALURONIC ACID FCH-150" (Kikkoman Biochemifa Co., Ltd.), an average molecular weight of 1400 to 1800 kDa; a product name of "BIOHYALURONIC ACID SODIUM SZE" (Shiseido Co., Ltd.), an average molecular weight of 1100 to 1600 kDa; a product name "BIOHYALURONIC ACID SODIUM HA 20N" (Shiseido Co., Ltd.), an average molecular weight of 1900 to 2700 kDa; a product name of "HYALURONIC ACID FCH-120" (Kikkoman Biochemifa Co., Ltd.), an average molecular weight of 1000 to 1400 kDa; a product name "HYALURONIC ACID HA-LQ" (Kewpie Corp.), an average molecular weight of 600 to 1200 kDa; a product name of "HYALURONIC ACID FCH-80" (Kikkoman Biochemifa Co., Ltd.), an average molecular weight of 600 to 1000 kDa; a product name "HYALURONIC ACID FCH-60" (Kikkoman Biochemifer Co., Ltd.), an average molecular weight of 500 to 700 kDa; a product name "HYALURONIC ACID FCH-SU" (Kikkoman Biochemifer Co., Ltd.), an average molecular weight of 50 to 110 kDa; a product name "HYALURONIC ACID (L)" (FAP Japan Co., Ltd.), an average molecular weight of 50 kDa or less; a trade name of "HYALUOLIGO" (Kewpie Corp.), an average molecular weight of 10 kDa or less; and a product name "HYALURONIC ACID (SL)" (FAP Japan) an average molecular weight of 10 kDa or less. Examples of the derivative of hyaluronic acid include products of a product name "SODIUM ACETYLATED HYALURONATE" (Shiseido Co., Ltd.), an average molecular weight of 10 to 100 kDa; a product name "HYALOVEIL" (Kewpie Corp.), an average molecular weight of 500 to 800 kDa,; a product name "HYALOREPAIR" (Kewpie Corp.), an average molecular weight of 10 kDa or less; and a product name "HYALUSION" (Kikkoman Biochemifer Co., Ltd.). The cosmetic composition of the present invention may make use of a hyaluronic acid having an especially small molecular weight, which is called, for example, nanosized hyaluronic acid or permeation type hyaluronic acid.

The content of hyaluronic acid or the analog thereof in the cosmetic composition of the present invention is not particularly limited as far as the advantageous effects of the present invention are produced. The content is usually from 0.0001 to 2%, preferably from 0.0005 to 1%, more preferably from 0.001 to 0.5% by weight of a total of the composition.

Examples of the pearl gloss imparting agent include ethylene glycol distearate, ethylene glycol monostearate, and triethylene glycol distearate

Examples of the conditioning agent include cationized cellulose, cationized starch, cationized fenugreek gum, cationized guar gum, cationized tara gum, cationized locust bean gum, cationized xanthan gum, diallyl quaternary ammonium salt/acrylamide copolymer, polyquaternium, vinylimidazolium trichloride/vinyl pyrrolidone copolymer, hydroxyethylcellulose/dimethyldiallylammonium chloride copolymer, vinylpyrrolidone/quaternized dimethylaminoethyl methacrylate copolymer, polyvinylpyrrolidone/alkylamino acrylate copolymer, polyvinylpyrrolidone/alkylaminoacrylate/vinylcaprolactam copolymer, vinylpyrrolidone/methacrylamidopropyltrimethylammonium chloride copolymer, alkylacrylamide/acrylate/alkylaminoalkylacrylamide/polyethy lene glycol methacrylate copolymer, and adipic acid/dimethylaminohydroxypropylethylenetriamine copolymer.

Examples of the scrub agent includes apricot nucleus powder, almond shell powder, apricot nucleus powder, sodium chloride particles, olive nucleus powder, seawater dried product particles, candelilla wax, nutshell powder, cherry nucleus powder, coral powder, charcoal powder, hazelnut nucleus powder, polyethylene powder, and silicic anhydride.

Examples of the blood circulation promoter include acetylcholine, ichthammol, caffeine, capsaicin, chantharidal tincture, $\gamma$-oryzanol, ginger tincture, zingerone, cepharanthin, swertia herb extra, tannic acid, red paper tincture, tolazoline, tocopherol nicotinate, and benzyl nicotinate.

Examples of the astringent component include zinc sulfate, hydroxyaluminum, aluminum chloride, zinc sulfocarbolate, and tannic acid.

Examples of the ultraviolet absorbing component include octyltriazone, hexyl diethylaminohydroxybenzoylbenzoate, octyl dimethoxybenzylidenedioxoimidazolidinepropionate, 2-ethylhexyl p-methoxycinnamate, t-butylmethoxydibenzoylmethane, phenylbenzimidazolesulfonic acid, octyl methoxycinnamate, and ethylhexyl methoxycinnamate.

Examples of the ultraviolet scattering agent include water-containing silicic acid, zinc silicate, cerium silicate, titanium silicate, zinc oxide, zirconium oxide, cerium oxide, titanium oxide, iron oxide, silicic anhydride and other inorganic compounds; substances each yielded by coating any one of these inorganic compounds with an inorganic powder of, for example, water-containing silicic acid, aluminum hydroxide, mica or talc, or by complexing any one of the inorganic compounds into resin powder of, for example, polyamide, polyethylene, polyester, polystyrene or nylon; and substances each yielded by treating any one of the substances further with, for example, silicone oil, or fatty acid aluminum salt.

Examples of the cleaning component include potassium laurate, potassium myristate, potassium palmitate, potassium stearate, and other alkali metal salts; alkanolamide salts, amino acid salts, and other soaps; sodium cocoylglutamate, sodium cocoylmethyltaurine, and other amino-acid-based surfactants; sodium lauressulfate, other ether

sulfate ester salts; sodium lauryl ether acetate, and other ether carboxylic acid salts; sodium alkylsulfosuccinate, and other sulfosuccinic acid ester salts; coconut oil fatty acid monoethanolamide, coconut oil fatty acid diethanolamide, and other fatty acid alkanolamides; sodium laurylphosphate, sodium polyoxyethylene lauryl ether phosphate, and other monoalkyl phosphoric acid ester salts; coconut oil fatty acid amide propyldimethylaminoacetate betaine, lauryldimethylaminoacetate betaine, 2-alkyl-N-carboxymethyl-N-hydroxyethylimidazolinium betaine, laurylhydroxysulfobetaine, sodium laurylhydroxysulfobetaine and sodium lauroylamideethylhydroxyethylcarboxymethylbetaine hydroxypropylphosphate, and other betaine type amphoteric surfactants; and sodium laurylaminopropionate, and other aminoacid type amphoteric surfactants.

Examples of the antibacterial component include isopropylmethylphenol, chlorhexidine, benzalkonium chloride, acrinol, ethanol, benzethonium chloride, cresol, gluconic acid and derivatives thereof, povidone iodine, potassium iodide, iodine, triclocarban, triclosan, a photosensitive element 101, a photosensitive element 201, paraben, phenoxyethanol, 1,2-pentanediol, alkyldiaminoglycine hydrochloride, piroctone olamine, and miconazole.

The anti-inflammatory agent may be any one of steroidal anti-inflammatory agents, and non-steroidal anti-inflammatory agents. Specific examples thereof include dexamethasone valerate acetate, dexamethasone, prednisolone valerate acetate (prednisolone valeric acid ester and acetic acid ester), prednisolone acetate, prednisolone, hydrocortisone acetate, hydrocortisone, urfenmate, and bufexamac. However, the anti-inflammatory agent is not limited thereto. Additional examples thereof include diclofenac, pyroxicam, ε-aminocaproic acid, bromelain, serrapeptase, and semi-alkaline proteinase; and pharmaceutically acceptable salts thereof. When the steroidal anti-inflammatory agent is used as the anti-inflammatory agent, it is preferred to use an anti-drug steroid, such as prednisolone valeric acid ester and acetic acid ester (PVA), which shows pharmacological activity in an affected part coated with this steroid and is metabolized to a low-activity substance in the body.

Examples of the vitamin include dl-α-tocopherol, dl-α-tocopherol succinate, calcium dl-α-tocopherol succinate and other vitamin Es; riboflavin, flavin mononucleotide, flavinadenine dinucleotide, riboflavin butyrate, riboflavin tetrabutyrate, sodium riboflavin 5'-phosphate, riboflavin tetranicotinate, and other vitamin B2s; dl-α-tocopherol nicotinate, benzyl nicotinate, methyl nicotinate, β-butoxyethyl nicotinate; 1-(4-methylphenyl)ethyl nicotinate, and other nicotinic acid analogs; ascorbigen-A, ascorbic acid stearic acid ester, ascorbic acid palmitic acid ester, L-ascorbyl dipalmitate, and other vitamin Cs; methylhesperidin, ergocalciferol, cholecalciferol, and other vitamin Ds; phylloquinone, farnoquinone, and other vitamin Ks; γ-oryzanol, dibenzoylthiamine, dibenzoylthiamine hydrochloride; thiamine hydrochloride, thiamine cetyl hydrochloride, thiamine thiocyanate, thiamine lauryl hydrochloride, thiamine nitrate, thiamine monophosphate, thiamine lysine salt, thiamine triphosphoric acid salts, thiamine monophosphate phosphoric acid salts, thiamine monophosphate, thiamine diphosphate, thiamine diphosphate, thiamine diphosphate hydrochloride, thiamine triphosphate, thiamine triphosphate monophosphoric acid salts, and other vitamin B1s; pyridoxine hydrochloride, pyridoxine acetate, pyridoxal hydrochloride, 5'-pyridoxal phosphate, pyridoxamine hydrochloride, and other vitamin B6s; cyanocobalamin, hydroxocobalamin, deoxyadenosylcobalamin, and other vitamin B12s; folic acid, pteroyl glutamate, and other folic acid analogs; nicotinic acid, nicotinic acid amide, and other nicotinic acid analogs; calcium pantothenate, pantothenyl alcohol (panthenol), D-pantosine, D-pantethine, coenzyme A, pantothenyl ethyl ether and other pantothenic acid analogs; biotin, biocytin, and other biotin analogs; ascorbic acid, sodium ascorbate, dehydroascorbic acid, sodium ascorbate phosphate, magnesium ascorbate phosphate, and other ascorbic acid derivatives, which are vitamin Cs; and carnitine, ferulic acid, α-lipoic acid, orotic acid, and other vitamin-like effect factors.

Examples of the peptide or the derivative thereof include keratin-degraded peptide, hydrolyzed keratin, collagen, fish-derived collagen, atelocollagen, gelatin, elastin, elastin-degraded peptide, collagen-degraded peptide, hydrolyzed collagen, hydroxypropylammonium chloride hydrolyzed collagen, elastin-degraded peptide, conchiolin-degraded peptide, hydrolyzed conchiolin, silk-protein-degraded peptide, hydrolyzed silk, sodium lauroyl hydrolyzed silk, soybean-protein-degraded peptide, hydrolyzed soybean protein, wheat protein, wheat-protein-degraded peptide, hydrolyzed wheat protein, casein-degraded peptide, and acylated peptides (such as palmitoyl oligopeptide, palmitoylpentapeptide, and palmitoyltetrapeptide) .

Examples of the amino acids or the derivative thereof include betaine (trimethylglycine), proline, hydroxyproline, arginine, lysine, serine, glycine, alanine, phenylalanine, β-alanine, threonine, glutamic acid, glutamine, asparagine, aspartic acid, cysteine, cystine, methionine, leucine, isoleucine, valine, histidine, taurine, γ-aminobutyric acid, γ-amino-β-hydroxybutyric acid, carnitine, carnosine, and creatine.

Examples of the cell activating agent include γ-aminobutyric acid, ε-aminocaproic acid, and other amino acids; retinol, thiamine, riboflavin, pyridoxine hydrochloride, pantothenic acid, and other vitamins; glycolic acid, lactic acid, and other α-hydroxy acids; and tannin, flavonoid, saponin, and a photosensitive element 301.

The cosmetic composition of the present invention may contain water. From the viewpoint of producing the advantageous effects of the invention remarkably, the water content may be 10% or more by weight of a total of the cosmetic composition, and is preferably 20% or more, more preferably 30% or more, even more preferably 40% or more, even more preferably 50% or more, even more preferably 60% or more, even more preferably 70% or more by weight thereof. The water content is preferably 99% or less, more preferably 98% or less by weight of the total of the cosmetic

composition. The water content may be from 10 to 99% by weight of the total of the cosmetic composition, and is preferably from 20 to 99%, more preferably from 30 to 99%, even more preferably from 40 to 99%, even more preferably from 50 to 99%, even more preferably from 60 to 99%, even more preferably from 70 to 99% by weight of the total of the cosmetic composition.

[pH]

The pH of the cosmetic composition of the present invention may be appropriately set in accordance with the species of the component (A), the species and the contents of the other blending components, the formulation form and the use method of the cosmetic composition, and others, and is not particularly limited as far as the pH is physiologically or pharmaceutically allowable. The pH may be, for example from 2 to 9. From the viewpoint of producing the advantageous effects of the present invention stably, the pH of the cosmetic composition of the invention is preferably from 2 to 8, more preferably from 2 to 7, even more preferably for 4 to 7, even more preferably from 4.5 to 6.5, even more preferably from 5.0 to 6.5.

[Viscosity]

The cosmetic composition of the present invention preferably has an appropriate viscosity since the composition is easily spread when applied to the skin. From such a viewpoint, the viscosity (at 25°C) of the cosmetic composition of the invention is preferably from about 1 mPa•s to 1000 Pa•s, more preferably from about 1 mPa•s to 500 Pa•s, even more preferably from 1 mPa•s to 100 Pa•s although the viscosity is not particularly limited. The cosmetic composition can be rendered a cosmetic composition having such a viscosity by selecting mainly the species and the use amount of the viscosity adjuster appropriately.

The viscosity (at 25°C) herein is according to a viscosity measuring method described in a general test method of the Japanese Pharmacopoeia seventeenth edition, and the viscosity denotes viscosity measured by a single cylindrical rotary viscometer (Brookfield type viscometer). Specifically, the viscosity denotes a value measured using a product TV-10M (Toki Sangyo Co., Ltd.). Its rotor, the rotational speed and other conditions are selected in accordance with a manual of the present instrument to measure the viscosity at 25°C.

The description of the single cylindrical rotary viscometer will be made hereinafter. The single cylindrical rotary viscometer is a viscometer for measuring the torque of a liquid when a cylinder in the liquid is rotated at a constant angular velocity. An instrument constant $K_B$ of the viscometer is experimentally determined by using a standard liquid for calibration of the viscometer in advance, and then the viscosity $\eta$ of the liquid is calculated by the following expression:

$$\eta = K_B \times T/\omega,$$

in which

- $\eta$ : liquid viscosity (mPa•s),
- $K_B$: instrument constant (rad/cm$^3$) ,
- $\omega$: angular velocity (rad/s), and
- T: torque ($10^{-7}$N•m) acting on the cylindrical surface.

[Formulation Form]

The cosmetic composition of the present invention can be prepared by blending the component (A) and the component (B) into each other and optionally blending other components into the blend, and making the resultant into a form that may be of various types in a usual way. The formulation form can be any dosage form suitable for applying the composition to the skin. Specifically, the formulation form is, for example, a lotion, a gel agent, a mist agent, an emulsion agent, a cream agent, an ointment agent, a mousse agent, a sheet agent (substrate-carried agent), an aerosol agent, or a spray agent. These formulations can be prepared by a method commonly used by those skilled in the art, and can be produced in accordance with, for example, a method described in Formulation General Rule of Japanese Pharmacopoeia No. 17. The formulation form is preferably a lotion agent, a gel agent, a mist agent or a sheet agent (substrate-carried agent) since the agent is excellent in use-feeling when applied to the skin. When the formulation contains an oily base agent and an aqueous base agent as in a cream agent or ointment agent, the formulation may be a W/O type or an O/W type and is more preferably an O/W type.

[Use]

Specific examples of an article to which the cosmetic composition of the present invention is applied include skin lotion, emulsion, gel, cream, beauty liquid, sunscreen cosmetic, pack, a mask, hand cream, body lotion, body cream, and other basic cosmetics; and face wash, cleansing (makeup remover), body shampoo, shampoo, rinse, hair treatment, and other cleansing cosmetics. The cosmetic composition may be rendered a cosmetic composition having both functions of basic and cleansing cosmetics, such as the so-called cleansing lotion, cleansing milk, or cleansing cream, which serves as both of skin lotion and cleansing.

The cosmetic composition of the present invention may be further made into a rise-off type, or leave-on type.

The cosmetic composition of the present invention is usable as an aqueous cleansing cosmetic material although the usage of the composition is not limited. About conventional cleansing agents, a large amount of an oil component is blended thereinto, so that after the agents are used, it is necessary to perform rinsing or wash the face to remove oils and sebum stains. Thereafter, it is further necessary to moisturize the skin with, for example, skin lotion or an emulsion to compensate for sebum components and others that are removed at the same time by the rising or the like. For example, when the present invention is used as an aqueous cleansing cosmetic material, the cosmetic material can be used as a leave-on type material. The makeup, the sebum stains and others can be removed only by wiping off these materials by cotton or the like after the use. The use of the wiping-type aqueous cleansing cosmetic material has come to be called water cleansing.

When the present invention is used for aqueous cleansing cosmetic materials, the cosmetic materials can singly attain a process from makeup removal through face washing, and skin care to moisture retention with skin lotion. Such a wiping-type aqueous cleansing cosmetic material is useful since the material responds to a need of performing cleansing in a short time without labor, and furthermore makeup removal can easily be attained even in an environment in which water is hard to use, or an environment in which rinsing and face washing are not easily performed in a sanitary manner. The wiping-type aqueous cleansing cosmetic material preferably contains a moisturizing component from the viewpoint of enhancing the material further in moisturizing function.

The cosmetic composition of the present invention contains polyoxyethylene polyoxypropylene glycol (component (A)), which is a pluronic-surfactant. Thus, the composition gives a mild stimulus to the skin and can be used for a person having a sensitive skin. In addition, the cosmetic composition of the present invention can be rendered a mild type cosmetic composition low in stimulus, which does not give excessively strong stimuli. When the cosmetic composition of the present invention is used as an aqueous cleansing cosmetic material of a leave-on type, the composition does not impose burden on the skin to be skin-friendly, or does not damage the moisture of the skin easily although the makeup removal performance of the composition is excellent.

When the present invention is used as an aqueous cleansing cosmetic material, the invention is usable for makeup removal of various cosmetic products such as foundation, shear foundation, lip, lip gloss, lip stains, lip liner, cheek rouge, face tint, cheek stains, cheek gel, cheek butter, eye shadow, eye blow powder, eye liner, mascara, bronzer, a facial brightening agent, a facial high-lighter, face powder, lotion, and coloring and moisturizing agents. Base makeup, eye makeup, and lip makeup cosmetics may contain a large amount of a cosmetic component and a highly water-resistant cosmetic component not to cause makeup collapse easily with moistures such as tears and sweat although the form of the makeup cosmetics is not limited. The cosmetic composition of the present invention is skin-friendly without imposing any burden on the skin, or does not damage the moisture of the skin easily although the cosmetic composition is excellent in makeup removing performance; thus, the composition is useful for the removal of base makeup, eye makeup and lip makeup cosmetics, and face washing, skin care, and moisturizing.

Eye makeup is applied to the vicinity of delicate eyes. Various cosmetic products, such as eye shadow, eye liner and mascara, are applied thereto in an overlap form to adjust the impression of the eyes. In some cases, eyelash-extensions or false eyelashes are applied to the eye vicinity. On the basis of, for example, the complexity of the structure of the eye vicinity, the skin of the eye vicinity may be made rough by excessive makeup removal, or the eyes, the skin of the eye vicinity or eyelashes may be made insanitary by insufficient makeup removal. The cosmetic composition of the present invention is useful for makeup removal of eye makeup, and for face washing, skin care, and moisture retention since the composition does not impose burden on the skin to be skin-friendly, or does not damage the moisture of the skin easily although the composition is excellent in makeup removal performance. Although the usage of the cosmetic composition of the invention is not limited, the composition is excellent in makeup removal performance not to need excessive rubbing washing of the composition. Consequently, for example, eyelash-extension or false eyelashes are not easily out of place. Thus, the composition is useful.

[Container]

The cosmetic composition of the present invention is usable in the state of being stored in a container having a shape and material selected appropriately in accordance with the use purpose and usage thereof. Examples of the

container shape include bottle type, tube type, jar type, dropper type, dispenser type, stick type, pouch bag, and Chiapak shapes. Examples of the material include polyethylene terephthalate, polypropylene, polyethylenes (such as HDPE, LDPE, and LLDPE)), ABS resin, ethylene vinyl alcohol resin, polystyrene, glass, and metals (such as aluminum). These materials are usable as the material of the container in the state of being subjected to various coating treatments, or in a combination form that is yielded, for example, by mixing two or more of the materials or in a lamination form, considering the strength, flexibility, weather resistance, or stability of the components, and other factors. Those skilled in the art can select the respective diameters and materials of a nozzle and a formulation-melting/discharging moiety of the container to restrict the jetted-out amount of the formulation from the container, or reduce the adhesion thereof to the container.

[Producing Method]

The cosmetic composition of the present invention can be prepared by heating each component at 20 to 80°C, dissolving components while stirring at room temperature. The stirring method may be a known method. For example, the following machine is usable: a fan-type stirrer, a disperser, or a homo-mixer. The cosmetic composition of the invention can be yielded by cooling the mixture made uniform by the melting to room temperature. According to a formulation of the cosmetic composition, during cooling, a liquid, paste, or solid cleansing oil can be obtained.

[Method for Giving Cloudiness-Restraining Effect]

The present invention can provide a method for giving a cloudiness-restraining effect onto a cosmetic composition, which comprising incorporating,
component (A) polyoxyethylene polyoxypropylene glycol, and
component (B) an amino acid based surfactant having, in a single molecule thereof, two or more hydrophobic groups and two or more hydrophilic groups. As described above, when a cosmetic composition containing polyoxyethylene polyoxypropylene glycol (component (A)) added with other components, the component (A) may be damaged in stability and become cloud or precipitate. It has been verified that even in such a case, when an amino acid based surfactant having, in a single molecule thereof, two or more hydrophobic groups and two or more hydrophilic groups (component (B)) is caused to coexist with the component (A), the cosmetic composition can be restrained from becoming cloud/precipitating to be effectively improved in stability. In this method, the species of the components (A) and (B), the blend ratio therebetween, the species and the blend proportions of other components that may be blended therewith, the use form thereof, and other factors are the same as described above.
The present invention may be made into embodiments described below.
A cosmetic composition comprising

(A) polyoxyethylene polyoxypropylene glycol, and
(B) an amino acid based surfactant having, in a single molecule thereof, two or more hydrophobic groups and two or more hydrophilic groups;

the cosmetic composition wherein the component (A) has a number-average molecular weight of 1000 to 30000;
the cosmetic composition wherein propylene oxide units of the component (A) have an average polymerization degree of 5 to 100;
the cosmetic composition wherein ethylene oxide units of the component (A) have an average polymerization degree of 2 to 200;
the cosmetic composition wherein the component (A) is polyoxyethylene polyoxypropylene glycol (26 E.O.) (30 P.O.) (poloxamer 184), and/or polyoxyethylene polyoxypropylene glycol (25 E.O.) (30 P.O.);
the cosmetic composition wherein the content of the component (A) is from 0.01 to 50% by weight of a total of the cosmetic composition;
the cosmetic composition wherein at least one of the hydrophobic groups in the component (B) has an acyl group;
the cosmetic composition wherein each of the hydrophobic groups in the component (B) is an acyl group derived from a saturated or unsaturated fatty acid having 2 to 20 carbon atoms;
the cosmetic composition wherein each of the hydrophilic groups in the component (B) is at least one selected from the group consisting of a carboxyl group, a sulfonic acid group, a sulfate group and a phosphate group, and salts of these groups;
the cosmetic composition wherein the component (B) is a compound represented by the following formula (3):

[Formula 4]

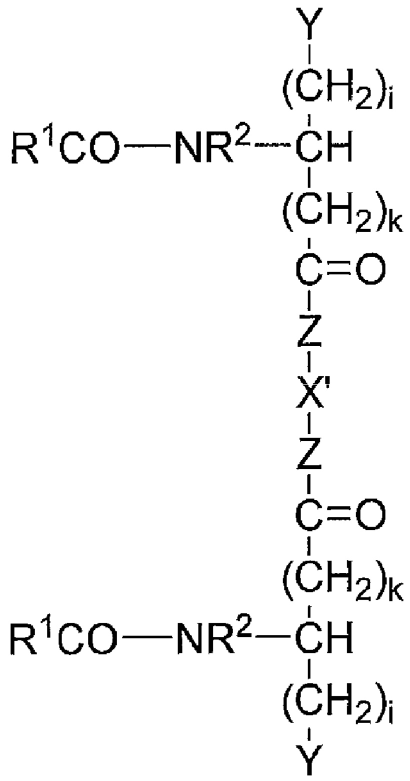

...(3)

wherein $R^1$ each are a hydrocarbon group having 1 to 23; $R^2$ each are hydrogen; X' is a hydrocarbon chain having a carboxyl group or a salt thereof and having 1 to 20 carbon atoms; Y each are a carboxyl group or a salt thereof; Z each are -NH-, and j and k each are zero or two, and j and k are not simultaneously zero;
the cosmetic composition wherein the component (B) is at least one selected from the group consisting of dilauramidoglutamide lysine and salts thereof;
the cosmetic composition wherein the content of the component (B) is from 0.01 to 30% by weight of a total of the cosmetic composition;
the cosmetic composition wherein about the blend ratio between the component (A) and the component (B), the total amount of the component (B) is from 0.001 to 210 parts by weight for 100 parts by weight of the component (A) ;
the cosmetic composition, further including a component (C) a pharmaceutically acceptable base agent and/or additive;
the cosmetic composition wherein the component (C) is at least one selected from the group consisting of antiseptic agents/preservatives, oxidation inhibitors, and anti-oxidants;
the cosmetic composition, further including water;
the cosmetic composition wherein the content of water is 10% or more by weight of a total of the cosmetic composition;
the cosmetic composition wherein the content of water is from 10 to 99% by weight of a total of the cosmetic composition;
the cosmetic composition, further including a moisturizing component;
the cosmetic composition wherein the moisturizing component is at least one selected from the group consisting of hyaluronic acid and analogs thereof, sodium chondroitin sulfate, collagen, and elastin;
the cosmetic composition wherein the hyaluronic acid and the analogs thereof each are at least one selected from the group consisting of hyaluronic acid, acetylated hyaluronic acid, hydrolyzed hyaluronic acid, cationized hyaluronic acid, and hyaluronic acid cross polymer; and salts of these compound;
the cosmetic composition wherein the composition is at least one selected from the group consisting of lotion agents, gel agents, mist agents, and sheet agents (substrate-carried agents);
the cosmetic composition wherein the composition is a leave-on type;
the cosmetic composition wherein the composition is used in the vicinity of an eye; and
the cosmetic composition wherein the composition is for makeup removal, face washing, skin care and moisturizing.

The present invention also relates to a method for giving a cloudiness-restraining effect to a cosmetic composition, comprising incorporating component (A) and component (B) into the cosmetic composition, wherein the component (A) is polyoxyethylene polyoxypropylene glycol; and the component (B) is an amino acid based surfactant having, in a

single molecule thereof, two or more hydrophobic groups and two or more hydrophilic groups. The present invention may also be a method for restraining a cosmetic composition from becoming cloud, comprising: incorporating component (A) and component (B) into the cosmetic composition, wherein the component (A) is a polyoxyethylene polyoxypropylene glycol; and the component (B) is an amino acid based surfactant having, in a single molecule thereof, two or more hydrophobic groups and two or more hydrophilic groups.

The present invention may also be use of component

(A) polyoxyethylene polyoxypropylene glycol, and component
(B) an amino acid based surfactant having, in a single molecule thereof, two or more hydrophobic groups and two or more hydrophilic groups for producing a leave-on type cosmetic composition.

Any one of the methods, or the use wherein the component (A) have a number-average molecular weight of 1000 to 30000;
any one of the methods, or the use wherein propylene oxide units of the component (A) have an average polymerization degree of 5 to 100;
any one of the methods, or the use wherein ethylene oxide units of the component (A) has an average polymerization degree of 2 to 200;
the cosmetic composition wherein the component (A) is polyoxyethylene polyoxypropylene glycol (26 E.O.) (30 P.O.) (poloxamer 184), and/or polyoxyethylene polyoxypropylene glycol (25 E.O.) (30 P.O.);
any one of the methods, or the use wherein the content of the component (A) is from 0.01 to 50% by weight of a total of the cosmetic composition;
any one of the methods, or the use wherein at least one of the hydrophobic groups in the component (B) has an acyl group;
any one of the methods, or the use wherein each of the hydrophobic groups in the component (B) is an acyl group derived from a saturated or unsaturated fatty acid having 2 to 20 carbon atoms;
any one of the methods, or the use wherein each of the hydrophilic groups in the component (B) is at least one selected from the group consisting of a carboxyl group, a sulfonic acid group, a sulfate group and a phosphate group, and salts of these groups;
any one of the methods, or the use wherein the component (B) is a compound represented by the following formula (3):

[Formula 5]

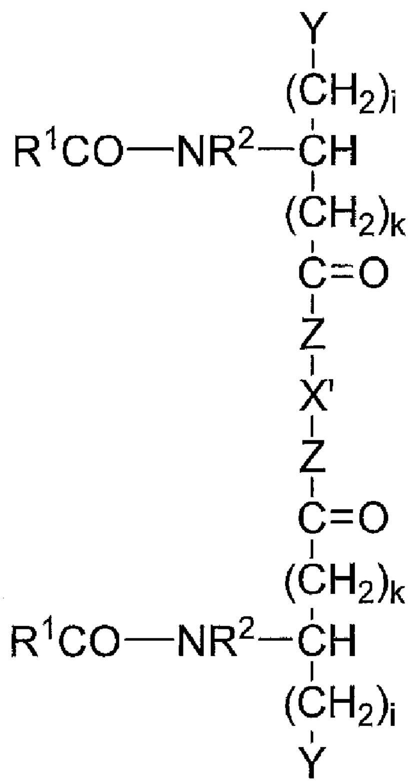

···(3)

in which $R^1$ each are a hydrocarbon group having 1 to 23; $R^2$ each are hydrogen; X' is a hydrocarbon chain having a carboxyl group or a salt thereof and having 1 to 20 carbon atoms; Y each are a carboxyl group or a salt thereof; Z each are -NH-, and j and k each are zero or two, and j and k are not simultaneously zero;
any one of the methods, or the use wherein the component (B) is at least one selected from the group consisting of dilauramidoglutamide lysine and salts thereof;
any one of the methods, or the use wherein the content of the component (B) is from 0.01 to 30% by weight of a total of the cosmetic composition;
any one of the methods, or the use wherein about the blend ratio between the component (A) and the component (B), the total amount of the component (B) is from 0.001 to 210 parts by weight for 100 parts by weight of the component (A) ;
any one of the methods, or the use, further including a component (C) a pharmaceutically acceptable base agent and/or additive;
any one of the methods, or the use wherein the component (C) is at least one selected from the group consisting of antiseptic agents/preservatives, oxidation inhibitors, and anti-oxidants;
any one of the methods, or the use, further including water;
any one of the methods, or the use wherein the content of water is 10% or more by weight of a total of the cosmetic composition;
any one of the methods, or the use wherein the content of water is from 10 to 99% by weight of a total of the cosmetic composition;
any one of the methods, or the use, further including a moisturizing component;
any one of the methods, or the use wherein the moisturizing component is at least one selected from the group consisting of hyaluronic acid and analogs thereof, sodium chondroitinsulfate, collagen, and elastin;
any one of the methods, or the use wherein the hyaluronic acid and the analogs thereof each are at least one selected from the group consisting of hyaluronic acid, acetylated hyaluronic acid, hydrolyzed hyaluronic acid, cationized hyaluronic acid, and hyaluronic acid cross polymer; and salts of these compound;
any one of the methods, or the use wherein the composition is at least one selected from the group consisting of lotion agents, gel agents, mist agents, and sheet agents (substrate-carried agents);
any one of the methods wherein the composition is a leave-on type;
any one of the methods, or the use, wherein the composition is used in the vicinity of an eye; and
any one of the methods, or the use wherein the composition is for makeup removal, face washing, skin care and moisturizing.

## EXAMPLES

Hereinafter, the present invention will be specifically described by way of formulation examples, working examples thereof, and others. However, the invention is not limited by the formulation example and the working examples.
Components used in the working examples are as follows:

Poloxamer 184: polyoxyethylene polyoxypropylene glycol (26 E.O.) (30 P.O.), PRONOUN #184, manufactured by NOF Corp.;
Polyoxyethylene polyoxypropylene glycol (25 E.O.) (30 P.O.): NEWPOL PE-64, manufactured by Sanyo Chemical Industries Co., Ltd.;
Sodium Dilauramidoglutamide Lysine: PELLICER L-30, manufactured by Asahi Kasei Finechem Co., Ltd.;
Sodium Cocoyl Glutamate: AMISOFT CS-22, manufactured by Ajinomoto Healthy Supply Co. Inc.;
Sodium Laureth Sulfate: EMAL E-27C, manufactured by Kao Corp.;
Lauryl dimethyl aminoacetate betaine: ANHITOLE 24B, manufactured by Kao Corp.;
Cocamide propyl betaine: GENAGEN CAB-818J, manufactured by Clariant (Japan) K.K.;
PEG-20 glyceryl triisostearate: EMALEX GWIS-320EX, manufactured by Nihon Emulsion Co., Ltd.;
Coconut oil fatty acid diethanolamide: AMIZOL CDE-G, manufactured by Kawaken Fine Chemicals Co., Ltd.; and
POE (40) sorbit tetraoleate: RHEODOLL 440V, manufactured by Kao Corp.

[Preparation Examples]

In accordance with a composition in Tables 1 and 2, individual components were stirred and dissolved while heated, to prepare each sample.

[Test Example 1: Storage Stability Test]

Fragments of each of the samples were filled into glass vessels (colorless and transparent; 20 mL), respectively,

and the vessels were stored at individual temperatures (25°C, 10°C, 4°C, and -0.5°C), respectively. After 12 hours, at the individual temperatures, the state of each of the sample fragments was visually observed.

(Evaluation Criterion)

○: The whole of a sample fragment is transparent, and no cloudiness is recognized therein.
Δ: Cloudiness is slightly recognized.
×: Cloudiness and/or precipitation is/are recognized. Results of the storage stability test are together shown in Tables 1 and 2.

[Table 1]

(Unit: % by weight)

| Components | | | | Example 1 | Example 2 | Comparative Example 1 | Comparative Example 2 | Comparative Example 3 |
|---|---|---|---|---|---|---|---|---|
| Composition | A | | Poloxamer 184 | 2 | - | 2 | 2 | 2 |
| | | | Polyoxyethylene polyoxypropylene glycol (25 E.O.)(30 P.O.) | - | 2 | - | - | - |
| | B | | Sodium Dilauramidoglutamide Lysine (30% solution in water) | 0.5 | 0.5 | - | - | - |
| | | Anionic surfactants | Sodium Cocoyl Glutamate (30% solution in water) | - | - | 0.5 | - | - |
| | | | Sodium Laureth Sulfate (30% solution in water) | - | - | - | 0.5 | - |
| | | Amphoteric surfactants | Lauryl dimethyl aminoacetate betaine (27% solution in water) | - | - | - | - | 0.5 |
| | | | Cocamide propyl betaine (30% solution in water) | - | - | - | - | - |
| | | Nonionic surfactants | PEG-20 glyceryl triisostearate | - | - | - | - | - |
| | | | Coconut oil fatty acid diethanolamide | - | - | - | - | - |
| | | | POE (40) sorbit tetraoleate | - | - | - | - | - |
| | | Salt | Sodium hydrogencarbonate | - | - | - | - | - |
| | C | Antiseptic agents | Methyl paraben | 0.2 | 0.2 | 0.2 | 0.2 | 0.2 |
| | | | Phenoxyethanol | 0.3 | 0.3 | 0.3 | 0.3 | 0.3 |
| | | | Purified water | Balance | Balance | Balance | Balance | Balance |
| | | | EDTA-2Na (disodium edetoate) | 0.05 | 0.05 | 0.05 | 0.05 | 0.05 |
| | | | DPG (dipropylene glycol) | 1 | 1 | 1 | 1 | 1 |
| | | | Glycerin | 1 | 1 | 1 | 1 | 1 |
| | | | Citric acid | 0.01 | 0.01 | 0.01 | 0.01 | 0.01 |
| | | | Disodium hydrogenphosphate | 0.05 | 0.05 | 0.05 | 0.05 | 0.05 |
| | Total | | | 100 | 100 | 100 | 100 | 100 |
| Test results | 25°C | | | ○ | ○ | ○ | ○ | ○ |
| | 10°C | | | ○ | ○ | × | × | △ |
| | 4°C | | | ○ | ○ | △ | △ | × |
| | -0.5°C | | | ○ | ○ | △ | ○ | × |

[Table 2]

(Unit: % by weight)

| Components | | | | Comparative Example 4 | Comparative Example 5 | Comparative Example 6 | Comparative Example 7 | Comparative Example 8 | Comparative Example 9 |
|---|---|---|---|---|---|---|---|---|---|
| Composition | A | | Poloxamer 184 | 2 | 2 | 2 | 2 | 2 | 2 |
| | | | Polyoxyethylene polyoxypropylene glycol (25 E.O.)(30 P.O.) | - | - | - | - | - | - |
| | B | | Sodium Dilauramidoglutamide Lysine (30% solution in water) | - | - | - | - | - | - |
| | | Anionic surfactants | Sodium Cocoyl Glutamate (30% solution in water) | - | - | - | - | - | - |
| | | | Sodium Laureth Sulfate (30% solution in water) | - | - | - | - | - | - |
| | | Amphoteric surfactants | Lauryl dimethyl aminoacetate betaine (27% solution in water) | - | - | - | - | - | - |
| | | | Cocamide propyl betaine (30% solution in water) | 0.5 | - | - | - | - | - |
| | | Nonionic surfactants | PEG-20 glyceryl triisostearate | - | 0.5 | - | - | - | - |
| | | | Coconut oil fatty acid diethanolamide | - | - | 0.5 | - | - | - |
| | | | POE (40) sorbit tetraoleate | - | - | - | 0.5 | - | - |
| | | Salt | Sodium hydrogencarbonate | - | - | - | - | 0.5 | - |
| | C | Antiseptic agents | Methyl paraben | 0.2 | 0.2 | 0.2 | 0.2 | 0.2 | 0.2 |
| | | | Phenoxyethanol | 0.3 | 0.3 | 0.3 | 0.3 | 0.3 | 0.3 |
| | | | Purified water | Balance | Balance | Balance | Balance | Balance | Balance |
| | | | EDTA-2Na (disodium edetoate) | 0.05 | 0.05 | 0.05 | 0.05 | 0.05 | 0.05 |
| | | | DPG (dipropylene glycol) | 1 | 1 | 1 | 1 | 1 | 1 |
| | | | Glycerin | 1 | 1 | 1 | 1 | 1 | 1 |
| | | | Citric acid | 0.01 | 0.01 | 0.01 | 0.01 | 0.01 | 0.01 |
| | | | Disodium hydrogenphosphate | 0.05 | 0.05 | 0.05 | 0.05 | 0.05 | 0.05 |
| | Total | | | 100 | 100 | 100 | 100 | 100 | 100 |
| Test results | 25°C | | | × | × | × | × | × | × |
| | 10°C | | | ○ | × | × | × | × | × |
| | 4°C | | | △ | △ | × | × | △ | △ |
| | -0.5°C | | | ○ | ○ | × | × | × | ○ |

As shown in Tables 1 and 2, the following new problem has been found out: when a component (A) that is a polyoxyethylene polyoxypropylene glycol coexists with a component that may be of various types in a cosmetic composition, the storage stability thereof is damaged so that at various storage temperatures, the composition generates cloudiness/precipitation (Comparative Examples 1 to 9). It has been demonstrated that in accordance with the component combined with the component (A), the storage stability of the component (A) is damaged in a low temperature range of -0.5 to 4°C (for example, Comparative Example 3), is damaged in a middle temperature range of 4 to 10°C (for example, Comparative Examples 1 and 2), is damaged in a normal temperature range of 10 to 25°C (for example, Comparative Examples 4 and 5), and is damaged in all the temperatures (for example, Comparative Examples 6 to 8). Thus, it is implied that stability decrease does not depend on temperature. This matter shows difficulty for designing formulations containing the component (A). Surprisingly, however, it has been found that when blending component (A) together with a component (B), the composition is restrained from becoming cloud/precipitating at any storage temperature from -0.5 to 25°C, so that the formulation stability of the composition is maintained. The mechanism showing such results is not limited, but is presumed as follows: the component (B) having a unique structure (amino acid based surfactant having, in a single molecule thereof, two or more hydrophobic groups and two or more hydrophilic groups) interacts with the component (A), and this interaction contributes to the stability of the whole of the cosmetic composition.

[Test Example 2: Storage Stability Test]

From Table 3 to 6, each sample was prepared same with formulation as in Example 1 except changing concentration of component (A) and component (B). The sample was then subjected to a storage stability test under the same conditions as in Test Example 1.

[Table 3]

| (Unit: % by weight) | | | | | | |
|---|---|---|---|---|---|---|
| Storage temperature: -0.5°C | | Component (A): Poloxamer 184 | | | | |
| | | 0.5 | 1 | 2 | 3 | 5 |
| Component (B) : PELLICER L-30 | 0.1(0.03) | Example 3 ○ | Example 4 ○ | Example 5 ○ | - | Example 6 ○ |
| | 0.3(0.09) | Example 7 ○ | Example 8 ○ | Example 9 ○ | - | Example 10 ○ |
| | 0.5(0.15) | Example 11 ○ | Example 12 ○ | Example 13 ○ | Example 14 ○ | Example 15 ○ |
| | 1(0.30) | Example 16 ○ | Example 17 ○ | - | - | Example 18 ○ |
| | 2(0.60) | - | Example 19 ○ | - | - | Example 20 ○ |
| | 3.4 (1.02) | Example 21 ○ | Example 22 ○ | - | - | Example 23 ○ |
| Note: About the component (B) content, a numerical value in each parenthesis pair shows the content of the component (B) as that of Sodium Dilauramidoglutamide Lysine (the same is applied to tables shown below).<br>Note: Any "-" in the table shows that no test was made (the same is applied to tables shown below). | | | | | | |

[Table 4]

| (Unit: % by weight) | | | | | | |
|---|---|---|---|---|---|---|
| Storage temperature: 4°C | | Component (A): Poloxamer 184 | | | | |
| | | 0.5 | 1 | 2 | 3 | 5 |
| Component (B) : PELLICER L-30 | 0.1(0.03) | Example 3 ○ | Example 4 ○ | Example 5 ○ | - | Example 6 ○ |
| | 0.3(0.09) | Example 7 ○ | Example 8 ○ | Example 9 ○ | - | Example 10 ○ |
| | 0.5(0.15) | Example 11 ○ | Example 12 ○ | Example 13 ○ | Example 14 ○ | Example 15 ○ |
| | 1 (0.30) | Example 16 ○ | Example 17 ○ | - | - | Example 18 ○ |
| | 2(0.60) | - | Example 19 ○ | - | - | Example 20 ○ |
| | 3.4(1.02) | Example 21 ○ | Example 22 ○ | - | - | Example 23 ○ |

[Table 5]

| (Unit: % by weight) | | | | | | |
|---|---|---|---|---|---|---|
| Storage temperature: 10°C | | Component (A): Poloxamer 184 | | | | |
| | | 0.5 | 1 | 2 | 3 | 5 |
| Component (B) : PELLICER L-30 | 0.5(0.15) | Example 11 ○ | Example 12 ○ | Example 13 ○ | Example 14 ○ | Example 15 ○ |
| | 1 (0.30) | Example 16 ○ | Example 17 ○ | - | - | Example 18 ○ |
| | 2(0.60) | - | Example 19 ○ | - | - | Example 20 ○ |
| | 3.4(1.02) | Example 21 ○ | Example 22 ○ | - | - | Example 23 ○ |

[Table 6]

| (Unit: % by weight) | | | | | | |
|---|---|---|---|---|---|---|
| Storage temperature: 25°C | | Component (A): Poloxamer 184 | | | | |
| | | 0.5 | 1 | 2 | 3 | 5 |
| Component (B) : PELLICER L-30 | 0.1(0.03) | Example 3 ○ | Example 4 ○ | Example 5 ○ | - | Example 6 ○ |
| | 0.3(0.09) | Example 7 ○ | Example 8 ○ | Example 9 ○ | - | Example 10 ○ |
| | 0.5(0.15) | Example 11 - | Example 12 ○ | Example 13 ○ | Example 14 ○ | Example 15 ○ |
| | 1(0.30) | Example 16 ○ | Example 17 ○ | - | - | Example 18 ○ |
| | 2(0.60) | - | Example 19 ○ | - | - | Example 20 ○ |
| | 3.4(1.02) | Example 21 ○ | Example 22 ○ | - | - | Example 23 ○ |

As shown in Tables 3 to 6, it has been verified that even when the component (A) and the component (B) are blended with each other at various contents thereof in a cosmetic composition, the cosmetic composition is restrained from becoming cloud/precipitating in a wide storage temperature range of -0.5 to 25°C to maintain formulation stability. The cosmetic composition of the present invention can be restrained from becoming cloud/precipitating in a wide storage temperature range of -0.5 to 25°C, and can continuously exhibit an original function that the component (B) has.

[Production Examples]

A known technique was used to prepare a cosmetic composition in accordance with each of Formulation Examples 1 to 4. These cosmetic compositions contain the component (A) and the component (B) to be good in storage stability. Moreover, these cosmetic compositions can each be used as a leave-on type cosmetic material without being washed out after applied to the skin. After makeup or sebum stains are wiped off with cotton or the like, a basic cosmetic material (such as skin lotion, emulsion or beauty liquid) may be applied to the wiped-off site or a new makeup may be applied thereto.

(Formulation Example 1) Cleansing Lotion

| | |
|---|---|
| Poloxamer 184 | 2.0% by mass |
| Sodium Dilauramidoglutamide Lysine (30% solution in water) | 0.5% by mass |
| Concentrated glycerin | 1.0% by mass |
| Dipropylene glycol | 1.0% by mass |
| Methyl paraben | 0.2% by mass |
| Phenoxyethanol | 0.3% by mass |
| Disodium edetate | 0.1% by mass |
| Citric acid | 0.01% by mass |

(continued)

| | |
|---|---|
| Disodium hydrogenphosphate | 0.05% by mass |
| Perfume | 0.2% by mass |
| Purified water | Appropriate volume |
| Total | 100% by mass |

(Formulation Example 2) Cleansing Lotion

| | |
|---|---|
| Poloxamer 184 | 1.0% by mass |
| Sodium Dilauramidoglutamide Lysine (30% solution in water) | 0.3% by mass |
| Concentrated glycerin | 1% by mass |
| Dipropylene glycol | 5% by mass |
| Sodium hyarulonate | 0.05% by mass |
| Methyl paraben | 0.2% by mass |
| Phenoxyethanol | 0.3% by mass |
| Disodium edetate | 0.1% by mass |
| Citric acid | 0.01% by mass |
| Disodium hydrogenphosphate | 0.05% by mass |
| Perfume | 0.2% by mass |
| Purified water | Appropriate volume |
| Total | 100% by mass |

(Formulation Example 3) Cleansing Sheet

<Cleansing Cosmetic Material>

| | |
|---|---|
| Polyoxyethylene polyoxypropylene glycol (25 E.O.) (30 P.O.) | 2.0% by mass |
| Sodium Dilauramidoglutamide Lysine (30% solution in water) | 0.5% by mass |
| Concentrated glycerin | 1.0% by mass |
| Dipropylene glycol | 1.0% by mass |
| Methyl paraben | 0.2% by mass |
| Phenoxyethanol | 0.3% by mass |
| Disodium edetate | 0.1% by mass |
| Xanthan gum | 0.05% by mass |
| Citric acid | 0.01% by mass |
| Disodium hydrogenphosphate | 0.05% by mass |
| Perfume | 0.2% by mass |
| Purified water | Appropriate volume |
| Total | 100% by mass |

<Sheet Substrate>

Nonwoven fabric (50% of pulp and 50% of rayon)

One part by mass of the sheet substrate was impregnated with 4 parts by mass of the cleansing cosmetic material.

(Formulation Example 4) Cleansing Gel

| | |
|---|---|
| Poloxamer 184 | 2.0% by mass |
| Sodium Dilauramidoglutamide Lysine (30% solution in water) | 0.5% by mass |
| Concentrated glycerin | 2.0% by mass |
| 1,3-Butylene glycol | 1.0% by mass |
| Methyl paraben | 0.2% by mass |
| Phenoxyethanol | 0.3% by mass |
| Disodium edetate | 0.05% by mass |
| Carboxyvinyl polymer | 0.5% by mass |
| Hydroxyethylcellulose | 0.05% by mass |
| Sodium hydroxide | 0.05% by mass |
| Perfume | 0.2% by mass |
| Purified water | Appropriate volume |
| Total | 100% by mass |

## Claims

1. A cosmetic composition comprising:

   (A) polyoxyethylene polyoxypropylene glycol, and
   (B) an amino acid based surfactant having, in a single molecule thereof, two or more hydrophobic groups and two or more hydrophilic groups.

2. The cosmetic composition according to claim 1, wherein at least one of the hydrophobic groups in the component (B) has an acyl group.

3. The cosmetic composition according to claim 1 or 2, wherein each of the hydrophilic groups in the component (B) is at least one selected from the group consisting of a carboxyl group, a sulfonic acid group, a sulfate group, a phosphate group, and salts of these groups.

4. The cosmetic composition according to any one of claims 1 to 3, further comprising (C) a pharmaceutically acceptable base agent and/or additive.

5. The cosmetic composition according to claim 4, wherein the component (C) is at least one selected from the group consisting of antiseptic agents/preservatives, oxidation inhibitors, and anti-oxidants.

6. The cosmetic composition according to any one of claims 1 to 5, wherein a content of water is 10% or more by weight of a total of the composition.

7. The cosmetic composition according to any one of claims 1 to 6, wherein the composition is at least one selected from the group consisting of lotion agents, gel agents, mist agents, and sheet agents (substrate-carried agents).

8. The cosmetic composition according to any one of claims 1 to 7, wherein the composition is a leave-on type.

9. A method for giving a cloudiness-restraining effect to a cosmetic composition, comprising incorporating component (A) and component (B) into the cosmetic composition, wherein
   the component (A) is polyoxyethylene polyoxypropylene glycol, and
   the component (B) is an amino acid based surfactant having, in a single molecule thereof, two or more hydrophobic groups and two or more hydrophilic groups.

## INTERNATIONAL SEARCH REPORT

International application No.
PCT/JP2018/008766

A. CLASSIFICATION OF SUBJECT MATTER
Int.Cl. A61K8/86(2006.01)i, A61K8/02(2006.01)i, A61K8/44(2006.01)i, A61K8/55(2006.01)i, A61Q19/00(2006.01)i
According to International Patent Classification (IPC) or to both national classification and IPC

B. FIELDS SEARCHED
Minimum documentation searched (classification system followed by classification symbols)
Int.Cl. A61K8/86, A61K8/02, A61K8/44, A61K8/55, A61Q19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY/MEDLINE/EMBASE/BIOSIS (STN), Mintel GNPD

C. DOCUMENTS CONSIDERED TO BE RELEVANT

| Category* | Citation of document, with indication, where appropriate, of the relevant passages | Relevant to claim No. |
|---|---|---|
| A | JP 8-231381 A (NOEVIR CO., LTD.) 10 September 1996, claims, examples 1-6 (Family: none) | 1-9 |
| A | WO 2015/186582 A1 (MANDOM CORPORATION) 10 December 2015, claims, examples 1-6, formulation examples 1-2 & KR 10-2016-0103139 A & CN 106068118 A | 1-9 |
| A | JP 2000-355516 A (AJINOMOTO CO., INC.) 26 December 2000, claims, examples 31-32 (Family: none) | 1-9 |
| A | JP 2014-073991 A (FANCL CORPORATION) 24 April 2014, claims, formulation example 3 (Family: none) | 1-9 |
| A | JP 2010-275293 A (SHISEIDO CO., LTD.) 09 December 2010, claims, example 5, formulation example 2 & US 2012/0164092 A1:claims, example 5, formulation example 2 & WO 2010/125838 A1 & EP 2425811 A1 & KR 10-2010-0126855 A & CN 102046146 A | 1-9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 April 2018 (13.04.2018) | 24 April 2018 (24.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017007995 A
- JP 2000204025 A